(19) Europäisches Patentamt
European Patent Office
Office européen des brevets

(11) Numéro de publication: **0 022 041**
**B1**

(12) **FASCICULE DE BREVET EUROPEEN**

(45) Date de publication du fascicule du brevet: **12.09.84**

(51) Int. Cl.$^3$: **C 07 D 263/58**

(21) Numéro de dépôt: **80420069.9**

(22) Date de dépôt: **16.06.80**

(54) **Procédé de préparation de benzoxazolone.**

(30) Priorité: **20.06.79 FR 7916334**

(43) Date de publication de la demande:
**07.01.81 Bulletin 81/01**

(45) Mention de la délivrance du brevet:
**12.09.84 Bulletin 84/37**

(84) Etats contractants désignés:
**AT BE CH DE FR GB IT LI LU NL SE**

(56) Documents cités:

**BERICHTE DER DEUTSCHEN CHEMISCHEN GESELLSCHAFT, vol. 35, 1902, Berlin DE C.GRAEBE et al.: "Über die Hofman'sche Reaktion (Überführung der Amide in Amine), pages 2747-2752**

**Le dossier contient des informations techniques présentées postérieurement au dépôt de la demande et ne figurant pas dans le présent fascicule.**

(73) Titulaire: **RHONE-POULENC AGROCHIMIE
14-20, rue Pierre Baizet
F-69009 Lyon (FR)**

(72) Inventeur: **Rouy, Noel
55, Avenue du Petit Pont
F-77330 Lesigny (FR)**

(74) Mandataire: **Brachotte, Charles et al
RHONE-POULENC AGROCHIMIE BP 9163-09
F-69263 Lyon Cedex 1 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 022 041 B1

## Description

La présente invention concerne un procédé de préparation de benzoxazolone à partir de salicylamide.

La benzoxazolone a pour formule:

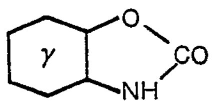

(I)

Il s'agit d'un produit intermédiaire utile pour la synthèse d'autres produits, par exemple l'insecticide connu sous le nom de phosalone. La benzoxazolone est quelque fois appelée, notamment en langue anglaise, benzoxazolinone.

Le salicylamide a pour formule:

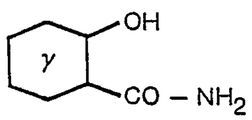

On a déjà préparé la benzoxazolone à partir de salicylamide en 1902 (Graebe et Rostovzeff, Ber. *35* p. 2747). Selon ce procédé, on fait réagir de l'hypochlorite en présence de soude sur de salicylamide. Cinquante ans plus tard (Arcus et Greenwood, J. Chem. Soc. 1953 p. 1937-1940), le même procédé a été confirmé et il est noté que de hautes concentrations en agent alcalin donnent de meilleurs résultats que les basses concentrations.

Un but de la présente invention est de permettre l'obtention de benzoxazolone à partir de salicylamide avec un rendement amélioré.

Un autre but de la présente invention est de permettre l'obtention de benzoxazolone à partir de salicylamide dans un milieu réactionnel assez concentré de manière à éviter le traitement de masses réactionnelles trop importantes.

Un autre but de l'invention est de permettre l'obtention de benzoxazolone à partir d'un milieu réactionnel assez fluide, de manière qu'il puisse être agité et homogénéisé convenablement.

D'autre buts et avantages de l'invention apparaîtront au cours de la description qui va suivre.

Il a maintenant été trouvé que ces buts pouvaient être atteints en tout ou partie grâce au procédé selon l'invention. Ce procédé est un procédé de préparation de benzoxazolone à partir de salicylamide, caractérisé en ce qu'on fait réagir le salicylamide avec des ions hypochlorite en présence d'eau et d'ions hydroxyle, d'ions sodium et d'ions potassium.

La réaction mise en oeuvre peut s'écrire schématiquement

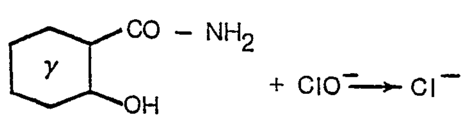


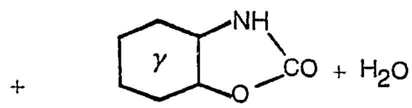


La concentration en ions hypochlorite dans le milieu réactionnel est généralement comprise entre 0,05 et 3 ion-g/l, de préférence comprise entre 0,1 et 1,5.

La concentration en ions hydroxyle dans le milieu réactionnel est généralement comprise entre 1,5 et 8 ion-g/l, de préférence comprise entre 2,5 et 6 ion-g/l.

La concentration en salicylamide dans le milieu réactionnel est généralement comprise entre 0,2 et 4 moles/l, de préférence comprise entre 0,5 et 2 moles/l.

Le rapport en nombre des ions $Na^+$ et $K^+$ présents est généralement tel que le rapport des concentrations d'ions $Na^+/K^+$, c'est à dire

$$\frac{Na^+}{K^+},$$

est compris entre 0,1 et 2, de préférence compris entre 0,3 et 1,5. La température de réaction est généralement comprise entre 0 et 50°C, de préférence comprise entre 10 et 30°C.

Un moyen commode de mise en oeuvre de la réaction consiste à mélanger une solution d'hypochlorite alcalin avec une solution alcaline de salicylamide.

On peut utiliser, par exemple, un hypochlorite de sodium ou de potassium; quant à la solution alcaline de salicylamide, il peut s'agir d'une solution dans la soude (NaoH) ou la potasse (KOH), mais selon un procédé préféré on mélange une solution aqueuse d'hypochlorite de sodium avec une solution aqueuse de salicylamide contenant de la potasse.

En fin de réaction, la benzoxazolone formée est isolée par tout moyen connu en soit. C'est ainsi qu'habituellement, on détruit l'excès d'hypochlorite par un agent réducteur, puis neutralise le milieu réactionnel (éventuellement chauffé pour être rendu plus fluide). Après neutralisation la benzoxazolone précipite et peut être recueillie par filtration ou essorage ou tout autre moyen équivalent.

Le salicylamide utilisé comme réactif dans la présente invention est un produit connu; il peut être mis en ceuvre tel quel, à l'état isolé, ou encore sous forme de solution ou de produit brut.

Il doit bien être entendu que dans l'exposé de la présente invention, on a indiqué que le procédé faisait réagir du salicylamide parce que tel est le produit mis en oeuvre le plus commodément; mais en réalité dans les conditions réactionnelles (ou préalablement) le salicylamide peut être sous forme salifiée (phénate alcalin substitué par un groupe CO $NH_2$); le mot salicylamide, dans le présent

exposé doit donc être compris comme incluant la forme phénate.

Les exemples suivants, donnés à titre non limitatif, illustrent l'invention et montrent comment elle peut être mise en oeuvre.

Exemple 1

A 330 $cm^3$ d'une solution aqueuse contenant 3,0 moles/l de salicylamide et 9,3 moles/l de potasse, on ajoute progressivement en 75 mn, 505 $cm^3$ d'une solution aqueuse contenant 2,17 moles/l d'hypochlorite de sodium et 2,2 moles/l de chlorure de sodium.

Le mélange est maintenu sous agitation et à 28°C pendant la coulée et encore 120 mn après la coulée. On ajoute alors 8 g de sulfite de sodium $Na_2SO_3$ (0,064 moles) pour détruire l'excès d'hypochlorite.

Ensuite on ajoute 230 $cm^3$ d'une solution aqueuse à 7,1 moles/l d'acide sulfurique tout en chauffant le mélange sous agitation à 70°C. On refroidit à 40°C puis on filtre.

Le précipité de benzoxazolone est lavé à l'eau. On obtient ainsi la benzoxazolone avec un rendement de 96% par rapport au salicylamide mis en oeuvre.

Exemple 2

On répète l'exemple 1 avec les modifications suivantes:

A 330 $cm^3$ d'une solution aqueuse contenant 3,0 moles/l de salicylamide et 9,3 moles/l de potasse, on ajoute, progressivement en 90 mn, 525 $cm^3$ d'une solution aqueuse contenant 2,10 moles/l d'hypochlorite de sodium et 2,2 moles/l de chlorure de sodium.

Le mélange est maintenu sous agitation et à 10°C pendant la coulée et encore 120 mn après la coulée.

On ajoute 11 g (0,087 mole) de sulfite de sodium. Ensuite, on ajoute 280 $cm^3$ d'une solution aqueuse à 11,5 moles/l d'acide chlorhydrique sous agitation à 25°C.

On filtre à température ambiante. Le précipité de benzoxazolone est lavé à l'eau.

On obtient la benzoxazolone avec un rendement de 95%.

Lorsqu'on répète les exemples 1 et 2 mais en utilisant soit uniquement des dérivés de $Na^+$, soit uniquement des dérivés de $K^+$, le milieu réactionnel devient très difficilement agitable par suite de formation abondante de précipités salins ce qui nécessite une forte dilution à l'eau; en outre il se forme des mousses abondantes qui gênent aussi considérablement le déroulement de la réaction.

**Revendications**

1. Procédé de préparation de benzoxazolone à partir de salicylamide caractérisé en ce qu'on fait réagir le salicylamide (éventuellement sous forme phénate) avec des ions hypochlorite en présence d'eau et d'ions hydroxyle, d'ions sodium et d'ions potassium.

2. Procédé selon la revendication 1 caractérisé en ce que le rapport des concentrations molaires $Na^+/K^+$ est compris entre 0,1 et 2.

3. Procédé selon la revendication 2 caractérisé en ce que le rapport des concentrations molaires $Na^+/K^+$ est compris entre 0,3 et 1,5.

4. Procédé selon l'une des revendications 1 à 3 caractérisé en ce que le concentration des ions hypochlorite dans le milieu réactionnel est comprise entre 0,05 et 3 ions g/l.

5. Procédé selon la revendication 4 caractérisé en ce que la concentration des ions hypochlorite est comprise entre 0,1 et 1,5 ion g/l.

6. Procédé selon l'une des revendications 1 à 5 caractérisé en ce que la concentration en ions hydroxyle dans le milieu est comprise entre 1,5 et 8 ions g/l.

7. Procédé selon la revendication 6 caractérisé en ce que la concentration en ions hydroxyle dans le milieu réactionnel est comprise entre 2,5 et 6 ions g/l.

8. Procédé selon l'une des revendications 1 à 7 caractérisé en ce que la concentration en salicylamide dans le milieu réactionnel est comprise entre 0,2 et 4 moles/l.

9. Procédé selon la revendication 8 caractérisé en ce que la concentration en salicylamide est comprise entre 0,5 et 2 moles/l.

10. Procédé selon l'une des revendications 1 à 9 caractérisé en ce que la température du milieu réactionnel est comprise entre 0 et 50°C.

11. Procédé selon la revendication 10 caractérisé en ce que la température est comprise entre 10 et 30°C.

12. Procédé selon l'une des revendications 1 à 11 caractérisé en ce que on mélange une solution aqueuse d'hypochlorite de Na avec une solution aqueuse de salicylamide en présence de potasse.

**Patentansprüche**

1. Verfahren zur Herstellung von Benzoxazolon, ausgehend von Salicylsäureamid, dadurch gekennzeichnet, daß man das Salicylsäureamid (gegebenenfalls in Form des Phenats) zur Umsetzung mit Hypochloritionen in Gegenwart von Wasser und von Hydroxylionen, Natriumionen und Kaliumionen bringt.

2. Verfahren nach Anspruch 1, dadurch gekennzeichnet, daß das Verhältnis der molaren Konzentrationen $Na^+/K^+$ zwischen 0,1 und 2 liegt.

3. Verfahren nach Anspruch 2, dadurch gekennzeichnet, daß das Verhältnis der molaren Konzentrationen $Na^+/K^+$ zwischen 0,3 und 1,5 liegt.

4. Verfahren nach einem der Ansprüche 1 bis 3, dadurch gekennzeichnet, daß die Konzentration der Hypochloritionen in dem

Reaktionsmedium im Bereich zwischen 0,05 und 3 Ionen-g/l liegt.

5. Verfahren nach Anspruch 4, dadurch gekennzeichnet, daß die Konzentration der Hypochloritionen im Bereich zwischen 0,1 und 1,5 Ionen-g/l liegt.

6. Verfahren nach einem der Ansprüche 1 bis 5, dadurch gekennzeichnet, daß die Konzentration der Hydroxylionen in dem Medium im Bereich zwischen 1,5 und 8 Ionen-g/l liegt.

7. Verfahren nach Anspruch 6, dadurch gekennzeichnet, daß die Konzentration an Hydroxylionen in dem Reaktionsmedium im Bereich zwischen 2,5 und 6 Ionen-g/l liegt.

8. Verfahren nach einem der Ansprüche 1 bis 7, dadurch gekennzeichnet, daß die Salicylsäureamidkonzentration in dem Reaktionsmedium im Bereich zwischen 0,2 und 4 Mol/l liegt.

9. Verfahren nach Anspruch 8, dadurch gekennzeichnet, daß die Salicylsäureamidkonzentration im Bereich zwischen 0,5 und 2 Mol/l liegt.

10. Verfahren nach einem der Ansprüche 1 bis 9, dadurch gekennzeichnet, daß die Temperatur des Reaktionsmediums im Bereich zwischen 0 und 50°C liegt.

11. Verfahren nach Anspruch 10, dadurch gekennzeichnet, daß die Temperatur im Bereich zwischen 10 und 30°C liegt.

12. Verfahren nach einem der Ansprüche 1 bis 11, dadurch gekennzeichnet, daß man eine wäßrige Lösung von Natriumhypochlorit mit einer wäßrigen Lösung von Salicylsäureamid in Gegenwart von Kaliumhydroxyd mischt.

## Claims

1. A process for the preparation of benzoxazolone from salicylamide, which comprises reacting salicylamide (if appropriate in the phenate form) with hypochlorite ions, in the presence of water and of hydroxyl ions, sodium ions and potassium ions.

2. A process according to claim 1, wherein the ratio of the molar concentrations of $Na^+$ to $K^+$ is between 0.1 and 2.

3. A process according to claim 2, wherein the ratio of the molar concentrations of $Na^+$ to $K^+$ is between 0.3 and 1.5.

4. A process according to one of claims 1 to 3, wherein the concentration of hypochlorite ions in the reaction medium is between 0.05 and 3 g.ions/litre.

5. A process according to claim 4, wherein the concentration of the hypochlorite ions is between 0.1 and 1.5 g.ions/litre.

6. A process according to one of claims 1 to 5, wherein the concentration of hydroxyl ions in the medium is between 1.5 and 8 g.ions/litre.

7. A process according to claim 6, wherein the concentration of hydroxyl ions in the reaction medium is between 2.5 and 6 g.ions/litre.

8. A process according to one of claims 1 to 7, wherein the concentration of salicylamide in the reaction medium is between 0.2 and 4 moles/litre.

9. A process according to claim 8, wherein the concentration of salicylamide is between 0.5 and 2 mols/litre.

10. A process according to one of claims 1 to 9, wherein the temperature of the reaction medium is between 0 and 50°C.

11. A process according to claim 10, wherein the temperature is between 10 and 30°C.

12. A process according to one of claims 1 to 11, which comprises mixing an aqueous solution of Na hypochlorite with an aqueous solution of salicylamide, in the presence of potassium hydroxide.